**Europäisches Patentamt**

**European Patent Office**

**Office européen des brevets**

(11) Publication number: **0 083 586**

**B1**

(12) # EUROPEAN PATENT SPECIFICATION

(45) Date of publication of patent specification: **12.11.86**

(51) Int. Cl.⁴: **C 02 F 11/04,** C 12 M 1/00, C 12 P 5/02

(21) Application number: **82900511.5**

(22) Date of filing: **11.02.82**

(86) International application number: **PCT/SE82/00040**

(87) International publication number: **WO 82/02706 19.08.82 Gazette 82/20**

(54) APPARATUS FOR FERMENTATION OF ORGANIC MATERIAL.

(30) Priority: **11.02.81 SE 8100939**

(43) Date of publication of application: **20.07.83 Bulletin 83/29**

(45) Publication of the grant of the patent: **12.11.86 Bulletin 86/46**

(84) Designated Contracting States: **AT BE CH DE FR GB LI NL**

(56) References cited:
DE-C- 419 494
DE-C- 658 138
DE-C- 942 034
FR-A-1 367 006
FR-A-2 461 747
GB-A- 442 458

(73) Proprietor: **ALFA-LAVAL AB**
**Box 500**
**S-147 00 Tumba (SE)**

(72) Inventor: **WIKHOLM, Bert Stefan**
**Ottevägen 115**
**S-146 00 Tullinge (SE)**
Inventor: **CARLSSON, Torsten Lennart Teodor**
**Frejagatan 10 A**
**S-151 41 Södertälje (SE)**
Inventor: **ÖSTER, Ake Rafael**
**Gärdesvägen 19**
**S-147 00 Tumba (SE)**

(74) Representative: **Simpson, Ronald Duncan Innes et al**
**A.A.Thornton & Co. Northumberland House**
**303-306 High Holborn**
**London WCIV 7LE (GB)**

Courier Press, Leamington Spa, England.

**Description**

This invention relates to an apparatus for the fermentation of organic material, including a fermentor having an inlet for organic material at one end and an outlet for fermented organic material at the other end, a rotatable screw extending through the fermentor for conveying organic material from the inlet to the outlet, and means for heating the organic material, at least at the inlet end of the fermentor.

An apparatus of this kind is known from French Patent Specification 2461747, in which the fermentor and screw are inclined upwardly from the inlet to the outlet and heating is effected by a water pipe secured in the fermentor.

Another fermentor is known from German Patent Specification 942034, in which heating is effected by a resistance heater extending axially through the fermentor within a protective tubular sleeve.

German Patent Specification 658138 describes a heated mixer for organic waste in which a heated screw is arranged vertically in the centre of a tank so as to operate as a mixer but not as a conveyor screw.

The present invention is characterized in that the screw extends substantially horizontally through the fermentor and is arranged to break by rotation the mat that would otherwise be formed at and below the level of organic material in the fermentor;

and that the screw blade is hollow at the inlet end only of the fermentor for internal heating of the screw blade in the region of the inlet.

With this construction, the screw blade provides a large heat transfer area and because of its movement through the material, it ensures an improved heat transfer efficiency.

A further advantage of the invention lies in the following circumstance. When fermenting manure anaerobically, gas bubbles containing methane are developed. These lift solid particles to the manure surface, so that there is formed a solid mat. The good mixing, that the screw carries out, prevents the formation of this mat and assists release of gas bubbles from the particles to collect in the upper part of the fermentor. From there the gas can be discharged.

A further advantage is that the screw will efficiently prevent solid material from being accumulated in the fermentor, and in time plugging it. The screw inhibits the formation of short circuit flows, which could means lower efficiency in the fermentation process. As the heat requirement of the fermentation process normally prevails at the inlet end of the fermentor, where the organic material is fed, it is sufficient to heat the transport screw blades only at the inlet of the fermentor. Setting of the desired temperature, for instance 35°C for anaerobic fermentation, can be done by regulating the temperature and/or the flow of the medium that is used for heating, such as warm water.

A further improvement of the heating of the organic material can be obtained by the provision of one or more internally heated pipes extending parallel with the axis of the screw, and preferably rotatable with the screw. These pipes can be fastened to the screw at its periphery, serving to carry the screw.

It may be desirable to inoculate the material entering the fermentor with microorganisms to accelerate the start of the fermenting process. According to one further characterizing feature of the invention this can be achieved by designing the fermentor with a space, located outside the periphery of the screw, which stretches along the screw, which space allows recirculation of fermented material towards the material inlet of the fermentor. The recirculation is carried out automatically by returning part of the material, that has reached the gable of the fermentor at the outlet end for the material, moving it outside the screw periphery back towards the inlet side.

The invention will now be explained in more detail, reference being made to the enclosed drawing which shows, by way of example, an embodiment of the apparatus in question.

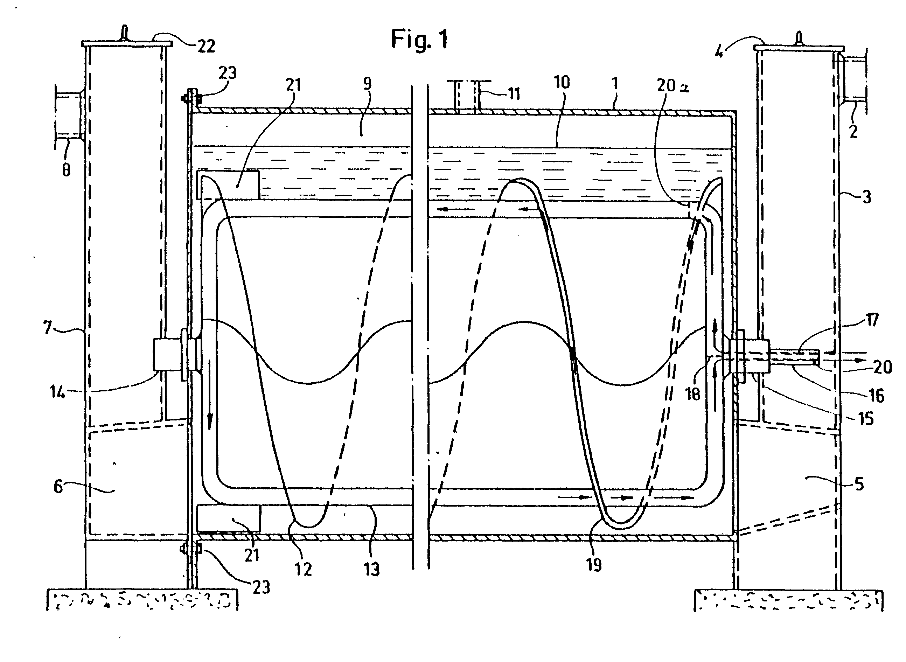

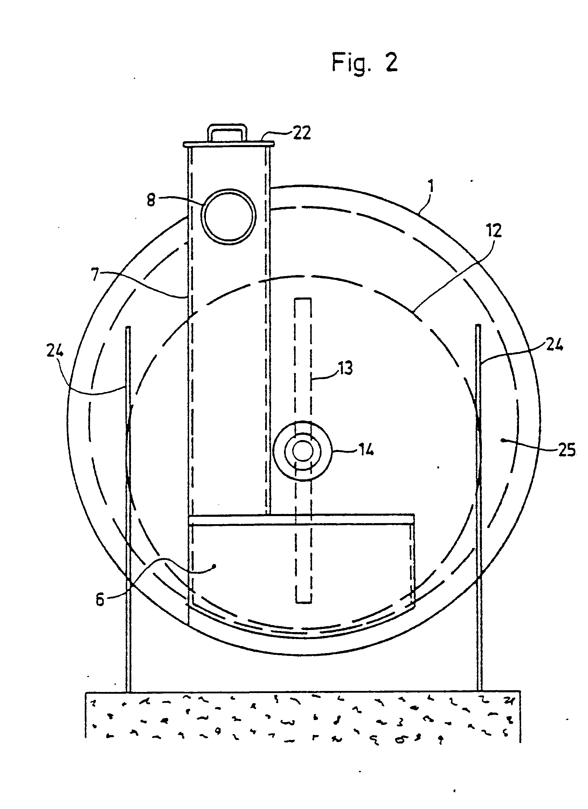

Figure 1 shows a longitudinal sectional view of the fermentor and Figure 2 a cross sectional view of the same apparatus, seen from the left.

Figure 1 just shows the opposite end portions of an elongated closed fermentor 1. This is provided with external heat insulation (not shown). Manure is fed through a pipe 2 to an inlet chamber 3, provided with a cap 4, in order that the inner parts of the chamber may be accessible if required. From a bottom space 5 in the chamber 3, manure flows further into the fermentor 1, and after having passed through the fermentor, into a bottom space 6 of an outlet chamber 7. Fermented manure then leaves this chamber through a weir outlet 8. In the fermentor there is formed methane gas, which fills a space 9 above the manure surface 10 in the fermentor 1. In the chambers 3 and 7 a manure level is established, which is higher than the level 10, by an amount determined by the gas pressure in the space 9. The developed methane gas is discharged under a certain overpressure through a pipe 11. A transport screw 12 is supported by a rectangular frame 13, consisting of pipes extending parallel with the axis of the screw 12, which frame is welded to the periphery of the screw. The frame is supported at the gables of the fermentor in bearings 14 and 15. The latter bearing is firmly connected to a shaft tap 16, connected to a motor (not shown) which rotates the frame 13 and thus also the screw 12. Hot water is fed through a channel 17, which goes through the shaft tap and the bearing 15. In the frame 13 there is provided a blocking wall 18, which in the illustrated position of the frame directs water upwardly through the frame. As indicated in the Figure the right hand end portion 19 of the blade of the screw 12, closest to the inlet, is hollow, and water can flow into this hollow blade part from the frame 13. A blocking wall 20a prevents water from flowing further to the left in the upper part of the frame.

As seen from Figure 1, the screw extends substantially horizontally through the fermentor, from the inlet end to the outlet end thereof.

As indicated in the Figure the cavity of the screw blade ceases after one blade turn, and the water then flows through a new connection once more into the frame 13 and continues through the frame to the blocking wall 18. Here the water reaches an outlet channel 20 and leaves the apparatus. The frame 13 is provided, at the outlet end, with baffles 21, which help to keep the outlet free and discharge the fermented manure into the space 6. Also the chamber 7 has a cap, denoted 22, which is removable to provide access to the inner parts of the chamber. The chamber 7 and the left gable of the fermentor are also for similar reasons arranged to be detachable from the rest of the fermentor by a screw joint 23.

In Figure 2, corresponding parts have the same reference numbers as in Figure 1. Furthermore, there are shown in Figure 2 two rods 24 which support the chamber 7. Furthermore, the Figure shows a space 25 between the periphery of the screw 12 and the inner side of the fermentor 1. Through this space part of the fermented manure is recirculated from the left gable of the fermentor to the right one, thus inoculating the fresh entering manure with microorganisms.

The rotation of the screw 12 and the frame 13 breaks the mat, that would otherwise be formed below the surface 10, especially through the flotating action, that the ascending gas bubbles perform on the solid particles of the manure.

The rotation of the screw and the frame also creates a large heat transfer area in relationship to the manure. This promotes the fermentation process, as an even and efficient heating of the organic material is achieved.

## Claims

1. Apparatus for the fermentation of organic material, including a fermentor having an inlet for organic material at one end and an outlet for organic material at the opposite end, a rotatable screw for carrying organic material from the inlet to the outlet, and means for heating the organic material characterized in that the screw (12) extends substantially horizontally through the fermentor (1) and is arranged to break by rotation the mat that would otherwise be formed at and below the level (10) of organic material in the fermentor;

and that the screw blade is hollow at the inlet end only of the fermentor for internal heating of the screw blade in the region of the inlet (3, 5).

2. Apparatus according to claim 1 characterized in that the organic material is additionally heated by one or more internally heated pipes (13) extending parallel with the axis of the screw (12) and preferably rotatable therewith.

3. Apparatus according to claim 2, wherein the or each said pipe (13) is positioned in the vicinity of the periphery of the screw (12).

4. Apparatus according to claim 1, 2 or 3, characterized in that a space (25) is left between the side wall of the fermentor (1) and the periphery of the screw (12) along the length thereof for recirculation of fermented material towards the inlet (3, 5) of the fermentor.

## Patentansprüche

1. Vorrichtung zum Fermentieren von organischem Material mit einem Fermenter mit einem Einlaß für organisches Material an einem Ende und einem Auslaß für organisches Material am entgegengesetzten Ende, einer drehbaren Schnecke zum Transport von organischem Material vom Ein- zum Auslaß, und einer Einrichtung zum Erwärmen des organischen Materials, dadurch gekennzeichnet, daß die Schnecke (12) im wesentlichen waagerecht durch den Fermenter (1) verläuft und so angeordnet ist, daß sie durch ihre Drehung die Matte aufbricht, die sich sonst an und unter der Oberfläche (10) des organischen Materials im Fermenter bilden würde, und daß die Schneckenklinge nur am Einlaßende des Fermenters hohl ausgeführt ist, um die Klinge im Bereich des Einlasses (3, 5) intern zu beheizen.

2. Vorrichtung nach Anspruch 1, dadurch gekennzeichnet, daß das organische Material zusätzlich durch ein oder mehrere innenbeheizte Rohre (13) erwärmt wird, die parallel zur Achse der Schnecke (12) verlaufen und vorzugsweise mit dieser drehbar sind.

3. Vorrichtung nach Anspruch 2, bei der das bzw. jedes der Rohre (13) nahe dem Umfang der Schnecke (12) angeordnet ist.

4. Vorrichtung nach Anspruch 1, 2 oder 3, dadurch gekennzeichnet, daß ein Raum (25) zwischen der Seitenwandung des Fermenters (1) und dem Umfang der Schnecke (12) entlang deren Länge belassen ist, um fermentiertes Material zum Einlaß (3, 5) des Fermenters zurückzuführen.

## Revendications

1. Appareil pour la fermentation d'un matériau organique, comportant une cuve de fermentation possédant une entrée pour un matériau organique à une extrémité et une sortie pour le matériau organique à l'extrémité opposée, une vis rotative pour transporter le matériau organique de l'entrée à la sortie, et des moyens pour chauffer le matériau organique, caractérisé en ce que la vis (12) s'étend sensiblement horizontalement à travers la cuve de fermentation (1) et est disposée de façon à briser par rotation le tampon qui, sinon, se formerait au niveau et en dessous du niveau (10) du matériau organique dans la cuve de fermentation;

et en ce que la pale de la vis est creuse uniquement à l'extrémité d'entrée de la cuve de fermentation pour le chauffage interne de la pale de la vis dans la région de l'entrée (3, 5).

2. Appareil selon la revendication 1, caractérisé en ce que le matériau organique est additionnellement chauffé par un ou plusieurs tuyaux chauffés intérieurement (13) s'étendant parallèlement à l'axe de la vis (12) et de préférence tournant avec celle-ci.

3. Appareil selon la revendication 2, dans lequel ledit tuyau ou chacun desdits tuyaux (13) est placé au voisinage de la périphérie de la vis (12).

4. Appareil selon la revendication 1, 2 ou 3, caractérisé en ce qu'un espace (25) est laissé entre la paroi latérale de la cuve de fermentation (1) et la prériphérie de la vis (12) le long de la longueur de celle-ci pour la recirculation du matériau fermenté vers l'entrée (3, 5) de la cuve de fermentation.

Fig. 1

0 083 586

Fig. 2

2